# EUROPEAN PATENT SPECIFICATION

(11) **EP 3 677 227 B1**
(45) Date of publication and mention of the grant of the patent: **27.04.2022**
(21) Application number: 18872418.1
(22) Date of filing: 02.11.2018
(51) Int. Cl.: A61F 2/90

(54) **STENT AND MEDICAL DEVICE COMPRISING SAME**
STENT UND EINEN STENT ENTHALTENE MEDIZINISCHE VORRICHTUNG
STENT ET DISPOSITIF MÉDICAL LE COMPRENANT

(30) Priority: 06.11.2017 JP 2017214041; 13.12.2017 JP 2017238831
(43) Date of publication of application: 08.07.2020
(73) Proprietor: EA Pharma Co., Ltd., Tokyo 104-0042 (JP); The Japan Wool Textile Co., Ltd., Kobe-shi, Hyogo 650-0037 (JP)
(72) Inventor: UESUGI, Shoji, Kakogawa-shi Hyogo 675-0053 (JP); SAOTOME, Toshiki, Kakogawa-shi Hyogo 675-0053 (JP); KINUGASA, Atsushi, Kato-shi Hyogo 673-1311 (JP); SUMIKAWA, Michito, Kawasaki-shi Kanagawa 210-8681 (JP); IIJIMA, Noriko, Kawasaki-shi Kanagawa 210-8681 (JP)
(74) Representative: Diehl & Partner
(86) International application number: PCT/JP2018/040825
(87) International publication number: WO 2019/088251

(56) References cited:
- GB-A- 2 470 484
- JP-A- 2004 528 115
- JP-A- 2009 160 079
- JP-A- 2012 527 321
- US-A1- 2011 264 186
- US-A1- 2011 264 186
- US-A1- 2015 100 133

## Description

### Technical Field

The present invention relates to a biodegradable stent for insertion into a tubular portion of a living body, such as the gastrointestinal tract, the bile ducts, the pancreatic duct, the blood vessels, the ureters, and the trachea, as well as a medical device including the stent.

### Background Art

Stenoses of bodily lumens are caused by various factors, and stent placement is often performed as the treatment. Typically, a stent in a collapsed state is loaded into a delivery system and delivered to a stenosis site, where the stent is expanded and placed. When expanding the stent, if the stent itself has no or an insufficient self-expanding force, it is necessary to expand the stent by inserting a balloon into the stent. Expanding a stent with the use of a balloon requires an operator to have skills so as to prevent lumen related injuries, and it is difficult to apply to a long stenosis or a stenosis having a bend. On the other hand, a self-expanding stent can be expanded simply by releasing the stent from the collapsed state at the stenosis site, and can be placed in a simple manner. Regarding the materials of self-expanding stents, metal stents using a shape-memory alloy such as a nickel-titanium alloy (nitinol) have conventionally been the mainstream and have already been applied to the gastrointestinal tract, including the esophagus, duodenum, large intestine, and the like, or to the bile ducts and the like. Most of the metal stents other than esophageal stents and the like that are relatively easy to remove after placement are placed in the body permanently. The reason for this is that serious complications such as lumen related injuries may be caused during stent removal. Therefore, metal stents for the gastrointestinal tract are mainly used for the treatment of stenoses associated with malignant tumors. However, in the case of benign gastrointestinal stenosis such as intestinal stenosis that occurs frequently in patients with Crohn's disease or ulcerative colitis, and postoperative stenosis that occurs after endoscopic submucosal dissection of an esophageal cancer, the placement of a metal stent, which is a foreign object, in the body for a long period of time increases the risk of perforation or the like caused by the stent moving, for example. Moreover, in the case of a stent for blood vessels, if an in-stent restenosis occurs due to a restenosis that occurs after the treatment with a metal stent, approaches that can be adopted for the treatment are limited. For these reasons, demands for a practicable biodegradable stent have been increasing recently.

Conventionally, polymers such as polylactic acid, polyglycolic acid, polydioxanone, and polycaprolactone, or copolymers thereof have been proposed as biodegradable materials for use in stents. However, these biodegradable polymers usually have a weaker expanding force compared with metal stents made of a nickel-titanium alloy (nitinol). Moreover, the expanding force is greatly affected by the type of the polymer that is used, the fiber diameter, the number of fibers that are used, and the manner in which a tubular body is braided, and therefore there are big challenges to overcome in order to adjust the expanding force to an expanding force that is suitable for the intended use of the stent. A method for increasing the expanding force is to increase the diameter of threads that are used and thereby increase the flexural modulus. For this purpose, with consideration given to the cross-sectional area of a stent loading portion in a delivery system, it is desirable to increase the diameter of threads that constitute a stent to an upper limit of the area that can be loaded. In order to load the stent into a delivery system, it is preferable that the threads are connected to each other at stent end portions, but if the threads are connected to each other, the connecting portions become larger than the threads that constitute the stent, and therefore, it is difficult to sufficiently increase the diameter of the threads that are used in the stent. Patent Document 1 proposes a biodegradable self-expandable stent, wherein the self-expanding properties are imparted based on the crossing angle between two sets of filaments that are used, the tensile strength of the filaments, and the like. Moreover, since stents are required to be ready to use whenever necessary and be clean, usually, a stent is loaded into a delivery system and is sterilized and stored in this state. However, in the case of a stent made of a polymer, there is a risk that, if the stent is stored for a long period of time in a collapsed state, the stent will deform, the expanding force thereof will decrease, and sufficient performance will not be achieved. Non-Patent Document 1 discloses cases in which benign stenoses of the small and large intestines were treated using biodegradable stents. The biodegradable stents that were used here were esophageal stents made of polydioxanone, but were not loaded into delivery systems in advance because the loading causes deformation. Patent Document 2 discloses a stent for a tubular path of a living body, wherein the recoverability after deformation and the deformation resistance of the stent were improved by arranging a plurality of reinforcement bars extending in the axial direction of a tubular body such that the reinforcement bars were distributed in the circumferential direction of the tubular body, and thereby suppressing elongation of the stent in the axial direction of the tubular body. However, this stent cannot be expected to have self-expanding properties, and is also difficult to collapse and load into a delivery system. According to Patent Documents 3 to 6, crossing points of threads at stent end portions are connected to each other to thereby prevent the threads from fraying, and the connecting portions are located in a single straight line in the circumferential direction. As a result of connecting the threads in this manner, the volume of the stent is larger than that when the threads are separate, causing a problem in that, when the stent is loaded into a delivery system, a spatial allowance is no longer generated at the stent end portions. Patent Document 7 proposes an arrangement of a single elastic thread into an annular knitted body. However, the tension of the elastic thread is non-uniform, causing a problem in that, when the stent is released from a delivery system, bending or distortion occurs in the stent Document JP2012527321 discloses an implantable medical device used for placement within a lumen of a patient that comprises a self-expanding tubular structure comprising a first polymer and a second polymer.

### Citation List

### Patent Documents

Patent Document 1: JP H11-57018A
Patent Document 2: JP 2009-160079A
Patent Document 3: JP 2002-200176A
Patent Document 4: JP 2004-528115T
Patent Document 5: JP 2015-155005T
Patent Document 6: JP 2016-146869A
Patent Document 7: WO 2016-035757

### Non-Patent Document

Non-Patent Document 1: "Biodegradable stents for the treatment of benign stenoses of the small and large intestines" Endoscopy 2012; 36: 833-839

### Disclosure of Invention

### Problem to be Solved by the Invention

Many of conventional self-expanding biodegradable stents are not subjected to end-portion processing, and are difficult to load into delivery systems. Moreover, even those subjected to the end-portion processing have problems in that the stents have high resistance during the release from delivery systems, end portions of the stents are unlikely to spread out after the release, and so on.

The present invention was made to address the above-described problems, and provides a stent in which connecting points at end portions of filament threads constituting the stent are offset and which is thereby easy to load into a delivery system and also facilitates the releasing operation, as well as a medical device including the stent. Furthermore, the present invention provides a biodegradable stent that has an enhanced expanding force and ensures that the end portions of the stent expand reliably, as well as a medical device including the biodegradable stent.

### Means for Solving Problem

A stent of the present invention is a stent formed by braiding a plurality of filament threads containing a biodegradable polymer into a cylindrical braid, wherein connecting points at end portions of the filament threads constituting the braid are arranged in two or more rows in a length direction of the braid.

Moreover, in the stent of the present invention, a plurality of elastic threads are further incorporated in the braid as warp threads and extend in the length direction, with both ends of the elastic threads being fixed to the braid.

Furthermore, in the stent of the present invention, at least one of the elastic threads is disposed outside at least a part of the stent and along at least a part of the stent in the length direction including the vicinity of either one of end portions of the stent; an end of the elastic thread is fixed to the vicinity of the end portion of the stent, and another end of the elastic thread is fixed to any portion of the stent; and in a state in which the stent is radially contracted, tension is applied to the elastic threads.

### Effects of the Invention

The stent of the present invention is formed by braiding a plurality of filament threads containing a biodegradable polymer into a cylindrical braid, and connecting points at end portions of the filament threads constituting the braid are arranged in two or more rows in the length direction of the braid. Thus, a stent that is easy to load into a delivery system and also facilitates the expanding operation is obtained. Moreover, the number of connecting points that are located at extreme end portions of the stent when the stent is collapsed can be reduced, and accordingly a spatial allowance is generated, making it possible to reduce the diameter of the end portions of the stent. This reduction in diameter provides the effects of making the stent easy to load into a delivery system and making it possible to reduce the force (opening force) that is necessary for the releasing operation. Moreover, it is easy for the end portions of the stent to spread out after the stent is released.

Also, according to the stent of the present invention, the expanding force of the stent in the length direction and the radial direction can be enhanced by incorporating a plurality of elastic threads extending in the length direction into the stent as warp threads. Moreover, the stent exhibits high deformation recoverability when released from the delivery.

Furthermore, according to the stent of the present invention, with the elastic threads being disposed outside the stent, a force that spreads the stent outward is exerted on the vicinities of the end portions of the stent due to the contracting forces of the elastic threads, and therefore, the end portions of the stent can be reliably expanded. In addition, since the elastic threads are fixed to the stent, the expansion can be achieved in an extremely simple manner without needing to use a special member.

### Brief Description of Drawings

[FIG. 1A] FIG. 1Ais a schematic diagram partially showing a flattened-out stent according to Embodiment 1 of the present invention.
[FIG. 1B] FIG. 1B is a photograph showing a lateral side of the stent in an expanded state.
[FIG. 1C] FIG. 1C is a photograph showing a lateral side of the stent when the stent is being released from a delivery system.
[FIG. 1D] FIG. 1D is a photograph showing a front side of the stent when the stent is being released from the delivery system, as seen from a released end side.
[FIG. 2A] FIG. 2Ais a schematic diagram partially showing a flattened-out stent according to Embodiment 2 of the present invention.
[FIG. 2B] FIG. 2B is a photograph showing a lateral side of the stent in an expanded state.
[FIG. 2C] FIG. 2C is a photograph showing a lateral side of the stent when the stent is being released from a delivery system.
[FIG. 2D] FIG. 2D is a photograph showing a front side of the stent when the stent is being released from the delivery system, as seen from a released end side.
[FIG. 3A] FIG. 3Ais a schematic diagram partially showing a flattened-out stent according to Embodiment 3 of the present invention.
[FIG. 3B] FIG. 3B is a photograph showing a lateral side of the stent in an expanded state.
[FIG. 3C] FIG. 3C is a photograph showing a lateral side of the stent when the stent is being released from a delivery system.
[FIG. 3D] FIG. 3D is a photograph showing a front side of the stent when the stent is being released from the delivery system, as seen from a released end side.
[FIG. 4] FIG. 4 is a schematic side view of a stent according to another embodiment of the present invention.
[FIG. 5A] FIG. 5Ais a schematic diagram partially showing a flattened-out conventional stent.
[FIG. 5B] FIG. 5B is a photograph showing a lateral side of the stent in an expanded state.
[FIG. 6] FIG. 6 is a schematic explanatory diagram showing a braid making apparatus.
[FIG. 7] FIG. 7Ais a schematic partial explanatory diagram illustrating the insertion of warp threads (elastic threads), of the braid making apparatus; and FIG. 7B is an operation diagram illustrating the movement of bobbins of the braid making apparatus.
[FIG. 8] FIG. 8 is a schematic side view of a delivery system in which the stent is loaded.
[FIG. 9] FIG. 9 is a schematic side view of a stent according to an embodiment of the present invention.
[FIG. 10] FIG. 10 is a schematic side view of a stent according to another embodiment of the present invention, in which a contact point where an elastic thread is in contact with a constituent thread of the stent is positioned at a distance of 1/2 of the stent length from an end portion of the stent.
[FIG. 11] FIG. 11 is a schematic side view of a stent according to yet another embodiment of the present invention, in which contact points where an elastic thread is in contact with constituent threads of the stent are positioned at a distance of 1/3 of the stent length from respective end portions of the stent, and the elastic thread between the contact points is disposed inside the stent.
[FIG. 12] FIG. 12 is a schematic side view of a stent according to yet another embodiment of the present invention, in which elastic threads are disposed outside the vicinities of end portions of the stent.
[FIG. 13] FIG. 13Ais a photograph showing a lateral side of a stent of Example 2 prior to the loading into a delivery system; FIG. 13B is a photograph showing a lateral side of the stent immediately after the release from the delivery system; and FIG. 13C is a photograph showing a lateral side of the stent three minutes after the release from the delivery system.
[FIG. 14] FIG. 14A is a photograph showing a lateral side of a stent of Example 3 prior to the loading into a delivery system; FIG. 14B is a photograph showing a lateral side of the stent immediately after the release from the delivery system; and FIG. 14C is a photograph showing a lateral side of the stent three minutes after the release from the delivery system.
[FIG. 15] FIG. 15 is a photograph showing an external appearance of a stent of Example 4 of the present invention prior to the placement of the stent.
[FIG. 16] FIG. 16 is a photograph showing an external appearance of the stent eleven minutes after the placement into the small intestine of a miniature pig in animal testing of Example 4 of the present invention.

### Summary

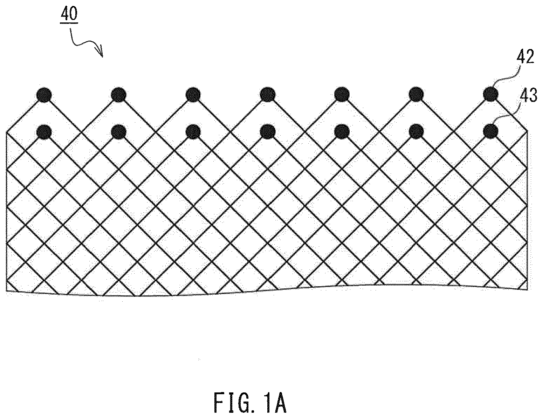

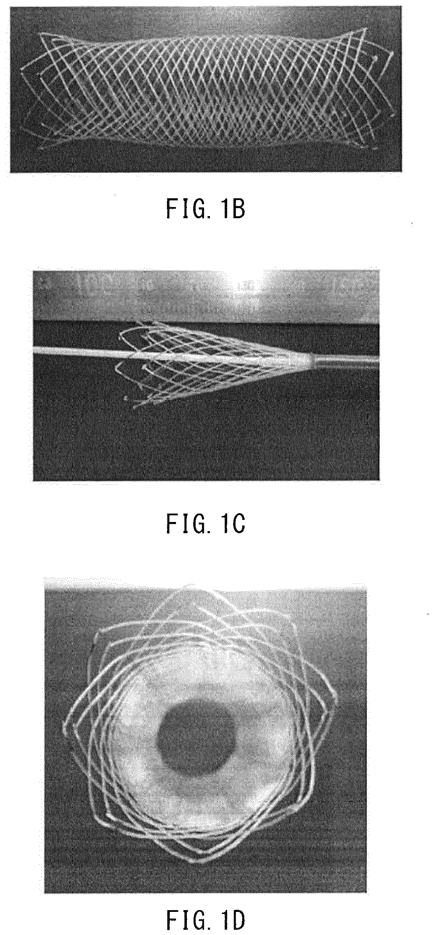

The invention relates to a stent as defined in claim 1. It also relates to a medical device as claimed in claim 13. Hereinafter, descriptions will be given using the drawings. In the drawings referenced below, identical reference numerals denote identical components. FIG. 1A is a schematic diagram partially showing a flattened-out stent 40 according to Embodiment 1 of the present invention; FIG. 1B is a photograph showing a lateral side of the stent in an expanded state; FIG. 1C is a photograph showing a lateral side of the stent when the stent is being released from a delivery system; and FIG. 1D is a photograph showing a front side of the stent when the stent is being released from the delivery system, as seen from a released end side. Connecting points 42 and 43 at each of which two threads constituting a braid are connected are arranged in two rows from an end portion of the braid in a length direction. With this configuration, the positions of the connecting points 42 and 43 when the stent 40 is collapsed are distributed into the two rows. Thus, the number of connecting points at the extreme end portion of the stent can be reduced by half. The other half of the connecting points are arranged in the next row that is inward of the row at the extreme end portion. As a result, a spatial allowance is generated at the extreme end portion, causing the stent to become thin when the stent is loaded into the delivery system, and hence facilitating the loading into the delivery system, and the expanding operation as well. Moreover, it is also possible to increase the diameter of the constituent threads, and the expanding force and the strength of the stent therefore can be increased. Regarding the method for connecting the threads at the connecting points, the connecting points can be formed through thermal fusion bonding, ultrasonic fusion bonding, joining with the use of an adhesive made of silicone or the like or a metal or resin tube, or the like. It can be seen from FIG. 1C that an end portion of the stent sufficiently spreads out after the stent release.

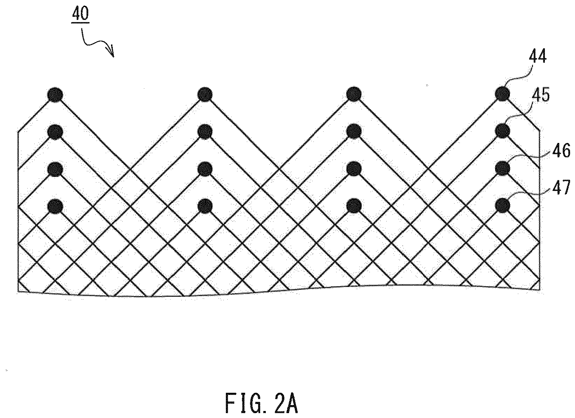

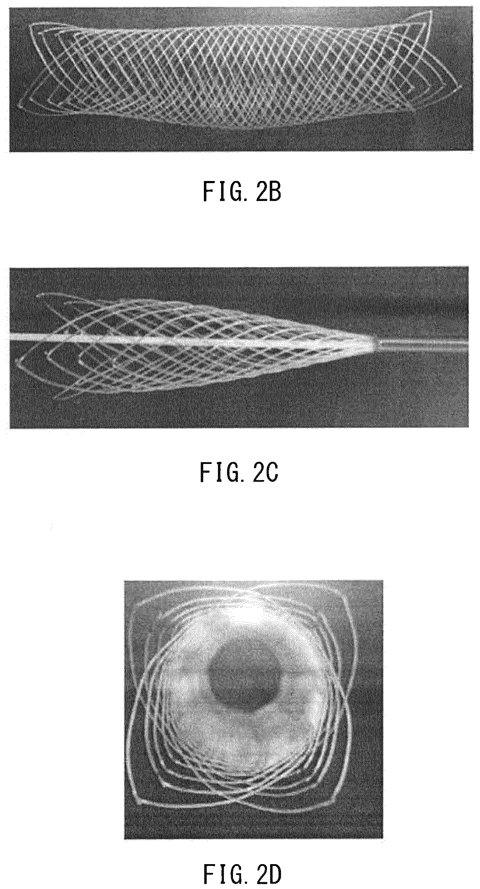

FIG. 2Ais a schematic diagram partially showing a flattened-out stent 40 according to Embodiment 2 of the present invention; FIG. 2B is a photograph showing a lateral side of the stent in an expanded state; FIG. 2C is a photograph showing a lateral side of the stent when the stent is being released from a delivery system; and FIG. 2D is a photograph showing a front side of the stent when the stent is being released from the delivery system, as seen from a released end side. Crossing points at each of which two threads constituting a braid cross each other are fused and fixed such that the crossing points are lined up in four rows at intervals in the length direction. Thus, connecting points 44 to 47 are lined up in four rows at intervals in the length direction. With this configuration, the number of connecting points that are located in a single row when the stent 40 is collapsed is reduced to 1/4, causing this portion to become thin when the stent is loaded into the delivery system, and hence facilitating the loading into the delivery system, and the expanding operation as well. Moreover, it is also possible to increase the diameter of the constituent threads, and the expanding force and the strength of the stent therefore can be increased. It can be seen from FIG. 2C that an end portion of the stent sufficiently spreads out after the stent release.

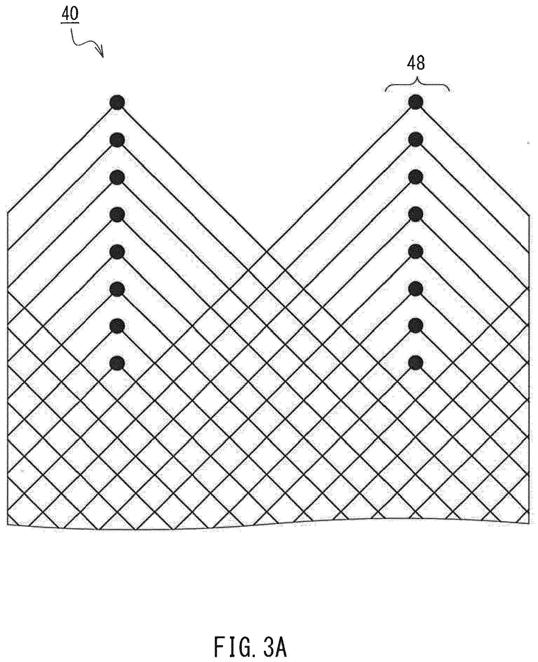

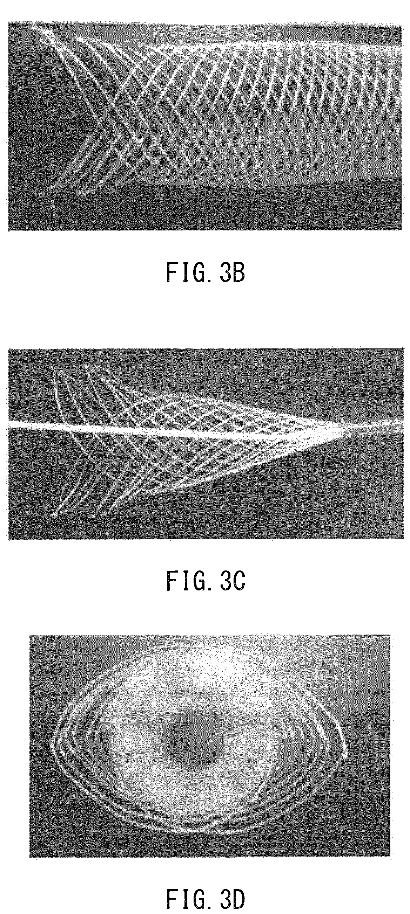

FIG. 3Ais a schematic diagram partially showing a flattened-out stent 40 according to Embodiment 3 of the present invention; FIG. 3B is a photograph showing a lateral side of the stent in an expanded state; FIG. 3C is a photograph showing a lateral side of the stent when the stent is being released from a delivery system; and FIG. 3D is a photograph showing a front side of the stent when the stent is being released from the delivery system, as seen from a released end side. Crossing points at each of which two threads constituting a braid cross each other are fused and fixed such that the crossing points are lined up in eight rows at intervals in the length direction. Thus, connecting points 48 are lined up in eight rows at intervals in the length direction. With this configuration, the number of connecting points that are located in a single row when the stent 40 is collapsed is reduced to 1/8, causing this portion to become thin when the stent is loaded into the delivery system, and hence facilitating the loading into the delivery system, and the releasing operation as well. Moreover, it is also possible to increase the diameter of the constituent threads, and the expanding force and the strength of the stent therefore can be increased. It can be seen from FIG. 3C that an end portion of the stent sufficiently spreads out after the stent release.

As in Embodiments 1 to 3 described above, the connecting points are arranged in two or more rows in the length direction of the stent, and the number of connecting points that can be lined up in the length direction depends on the number of filament threads constituting the stent, and is up to a number obtained by dividing the number of filament threads by 4. In terms of the extent to which the ease of the loading into a delivery system and the ease of the expanding operation are to be improved, as well as the uniformity of expansion of end portions when the stent is expanded, the number of connecting points that are lined up is preferably two to four rows. For this reason, among Embodiments 1 to 3, the stents of Embodiments 1 and 2 are more preferable.

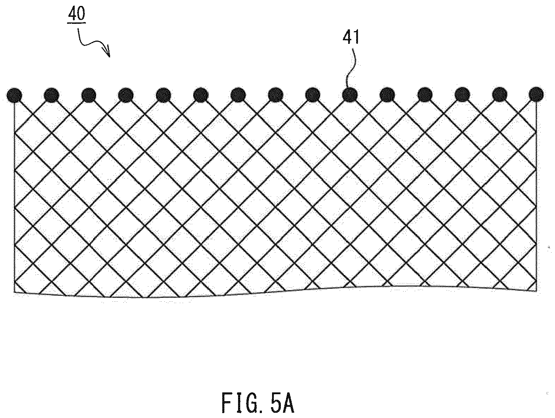

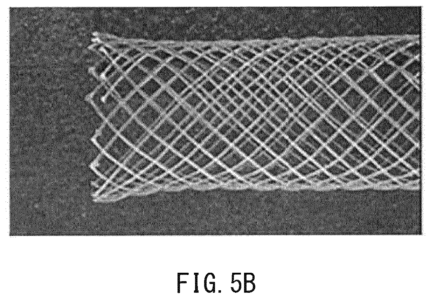

FIG. 5Ais a schematic diagram partially showing a flattened-out stent 40 that is constituted by a conventional braid; and FIG. 5B is a photograph showing a lateral side of the stent in an expanded state. All the crossing points of threads in the diameter direction are connected through thermal fusion bonding or the like, and thus, connecting points 41 are lined up side-by-side in a single row on the circumference. When the stent 40 is collapsed, the positions of all the connecting points 41 are on the same circumference, causing the stent to bulge and being likely to become an obstacle during the loading into the delivery system.

The stent of the present invention is formed by braiding a plurality of biodegradable filament threads into a cylindrical braid.

The filament threads constituting the braid may be monofilament threads or multifilament threads, and are preferably monofilament threads. Monofilament threads have a high degree of stiffness and are convenient for expanding a tubular portion of a living body.

The diameter of the filament threads is preferably 0.15 to 1.0 mm, more preferably 0.15 to 0.8 mm, and even more preferably 0.2 to 0.4 mm. With a filament thread diameter within the above-described range, the stent can retain a sufficient expanding force.

The number of filament threads constituting the braid is preferably 16 or greater, more preferably 24 or greater, and even more preferably 32 or greater. The upper limit is 64 or less. When the number of filament threads is within the above-described range, each filament thread constitutes the braid while having a spiral shape, and therefore, a stent with even higher self-expanding properties and deformation recoverability is obtained. For similar reasons, the braid angle of the filament threads (braiding threads) constituting the braid is preferably 30° to 80°. This angle is more preferably 35° to 75°, and even more preferably 45° to 65°. When the angle between the braiding threads is within the above-described range, a braid that has a complete cylindrical shape without distortion can be obtained. As used herein, the braid angle refers to an acute angle between the length direction of the braid as a whole and the direction of the braiding threads. Moreover, the number of braid intersections is 3 braid intersections/inch or greater, and preferably 4 to 18 braid intersections/inch. Thus, the thread density and the braid intersection density are high, the strength is also high, and the resilience is also high. Braiding patterns include round braiding and square braiding, and a braid that is braided by using round braiding is hollow and suitable for a stent.

A braiding machine is capable of varying mainly the thickness of braids depending on the number of carriers (number of filament threads that are used). When braiding a braid through a braiding process, it is possible to obtain a cylindrical braid by braiding a braid while vertically moving up and down (thrusting up) a metal or wooden bar from under the braid at a center portion of the braiding machine, the bar having a tip portion that has a circular or polygonal rounded shape with a size substantially equal to the inner diameter of the braid. Moreover, it is also possible to stabilize the shape of the braid by installing a heater between the thrust-up stage and a take-up stage and performing contactless heat treatment.

The polymer that composes the biodegradable filament threads is preferably at least one selected from biodegradable synthetic polymers, such as polyglycolic acid, poly-L-lactic acid, poly-D-lactic acid, polycaprolactone, polydioxanone, and polyethylene glycol, as well as their copolymers; and biodegradable natural polymers, such as collagen, gelatin, glycosaminoglycan, chitin, chitosan, hyaluronic acid, alginic acid, as well as silk fibroin, spider silk fibroin, and the like.

It is said that the degradation period of biodegradable synthetic polymers in a living body is about two weeks for a polyglycol, about six months for a polylactic acid thread, and one to two years for polycaprolactone, but the degradation period can be adjusted using the copolymer ratio, the polymer blend, the molecular weight, and the degree of crystallinity. Moreover, the degradation period of biodegradable natural polymers in a living body can be adjusted by using the molecular weight, performing structure control, imparting a crosslinked structure, and so on. In the stent of the present invention, the main portion of the braid is formed of the biodegradable filament threads, and therefore, the polymer will biodegrade within a predetermined period of time. In the case where the stent is used for the gastrointestinal tract, even when the stent includes a substance that is non-biodegradable, the substance will be excreted with the stool and hence cause no problems.

Preferably, any thread constituting the stent contains a substance that is X-ray detectable. For example, barium sulfate particles are mixed into a filament thread constituting the braid in advance. This makes it possible to accurately detect the location of the stent through X-ray irradiation from outside the living body and to also detect whether or not the stent has biodegraded. Alternatively, the detection may also be made possible by passing the filament threads constituting the braid through a radiopaque metal tube or coil made of platinum, platinum/palladium, platinum/iridium, platinum/tungsten, or the like.

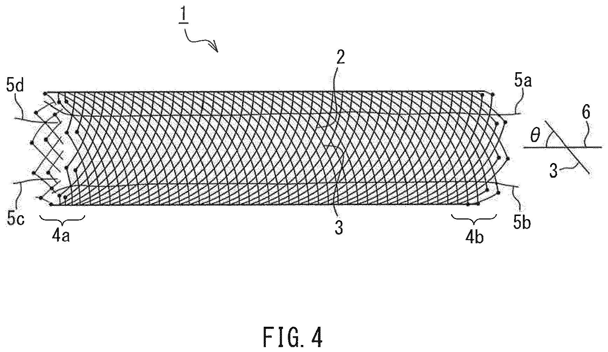

FIG. 4 is a schematic side view of a stent according to another embodiment of the present invention. A stent 1 is braided with braiding threads 2 and 3. In two end portions 4a and 4b, the threads are connected through fusion bonding or the like. As is the case with Embodiment 1, connecting points at each of which two threads constituting the braid are connected to each other are formed such that the connecting points are arranged in two rows in the length direction. Moreover, four warp threads 5a to 5d that are constituted by elastic threads and extend in the length direction are incorporated in the stent 1 through warp thread insertion, and when the stent 1 is expanded and allowed to stand with no load applied thereto, the warp threads 5a to 5d are in a contracted state. End portions of the warp threads are also fixed through fusion bonding. In this stent 1, the braiding threads are braided coarsely, and there are gaps. The braid angle of the braiding threads 2 and 3 is adjusted to a predetermined angle within a braid angle range of 30° to 80°.

Preferably, the two ends of the elastic threads are fixed to the braid. With this configuration, the expanding force of the stent in the length direction and the radial direction can be enhanced. Moreover, when released from a delivery system, the stent has high deformation recoverability. The plurality of elastic threads are preferably arranged such that two to six elastic threads are arranged at regular intervals around the circumference as seen in a cross section of the stent, and more preferably, three to five elastic threads are arranged at regular interval around the circumference of the stent as seen in a cross section. Preferably, the plurality of elastic threads are arranged at substantially equiangular positions as seen in a cross-sectional direction of the stent. When the plurality of elastic threads are uniformly arranged as seen in the cross-sectional direction, tension of the elastic threads that is necessary to cause the stent to self-expand is uniform, preventing the stent from bending after being released from the delivery system. Moreover, the ease of the loading of the stent into the delivery system is also favorable.

Rubbers, polyurethane threads, and thermoplastic elastomer threads that are biocompatible can be suitably used as the elastic threads. An example of the biocompatible polyurethane threads is a product manufactured under the brand name "Pellethane" by Lubrizol, U.S.A., which is USP Class VI approved. The polyurethane threads are preferably filament threads with a diameter of 50 to 500 pm, and more preferably filament threads with a diameter of 60 to 300 µm. The elastic threads may be incorporated through warp thread insertion during the production of a braid or may be attached to an outside of the produced braid.

The tension applied to the elastic threads in a state in which the stent is radially contracted is preferably 0.1 to 5.0 N/thread, more preferably 0.3 to 3.0 N/thread, and particularly preferably 0.5 to 2.5 N/thread. Here, N refers to Newton. This configuration ensures that the end portions of the stent expand more reliably.

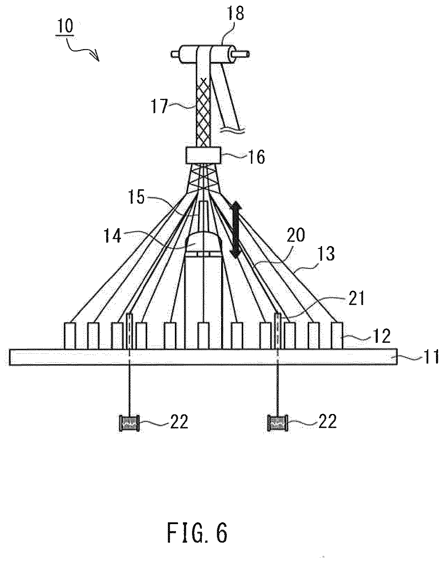

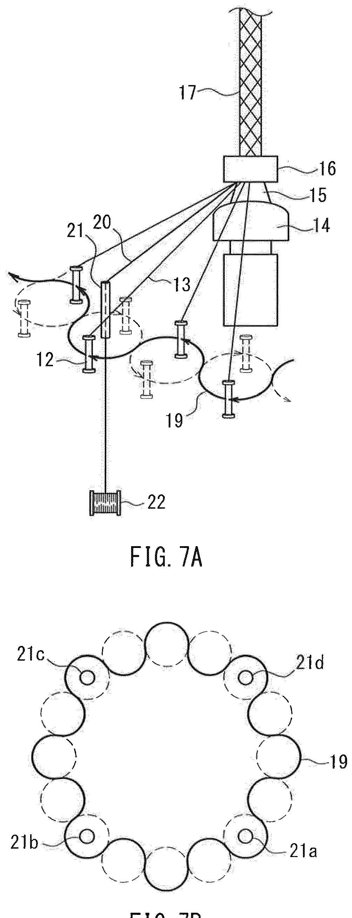

FIG. 6 is a schematic explanatory diagram showing an apparatus for making a round braid. This braid making apparatus 10 includes a mount 11, bobbins (carriers) 12, a mandrel 14, and a driving device, which is not shown. Bobbins 12 rotatively move on the solid lines indicating tracks 19 on the mount 11, thereby allowing threads 13 that are wound on the bobbins 12 to be braided over the mandrel 14 that performs thrust-up motions, and consequently, a braid 17 is produced. Warp threads 20 that are constituted by elastic threads are supplied from reels 22 that are disposed under the mount 11. A thrust-up portion 16 is composed of a hemispherical head that vertically moves up and down in conjunction with the rotative movement of the bobbins 12, as well as a cylinder portion 15 that is located at a center portion of the head and has a cylindrical shape (or a polygonal shape). The outer diameter of the cylinder portion 15 is substantially equal to the inner diameter of the braid 17. If necessary, the braid 17 is transferred to a heater and heat-set. The braid 17 goes around a take-up guide (pulley) 18 and is discharged down into a storage container. In the above-described configuration, the stroke length and the number of strokes of the mandrel 14 are set as appropriate. The braid may also be subjected to heat setting. The warp threads 20 as well as the reels 22 and pipes 21 for the warp threads 20 will be described with reference to FIGS. 7A and 7B.

FIG. 7Ais a schematic partial explanatory diagram illustrating the insertion of a warp thread (elastic thread) of the braid making apparatus; and FIG. 7B is an operation diagram illustrating the movement of the bobbins. In FIG. 7A, the warp thread 20 constituted by an elastic thread is supplied from the reel 22 disposed under the mount 11, and inserted into the braid from the pipe 21 located inside the track (rail) 19 on which bobbins of the braiding threads move. The pipe 21 is disposed at a position higher than the bobbins (carriers) 12 of the braiding threads, and the warp thread is inserted into the braid after passing through the pipe 21. The relationship between the tracks (rails) 19 and the pipes are as shown in FIG. 7B, and pipes 21a to 21d are uniformly arranged at the centers of respective tracks (rails) 19. Note that warp threads can also be inserted by hand without using a braid making apparatus such as the one described above.

In a stent of another embodiment of the present invention, the elastic threads are each disposed outside at least a part of the stent and extend in the length direction. Each elastic thread is disposed along at least a part of the stent in the length direction including the vicinity of either one of the end portions of the stent. One end of the elastic thread is fixed to the vicinity of the end portion of the stent, while the other end is fixed to any portion of the stent. Moreover, in a state in which the stent is radially contracted, tension is applied to the elastic threads. With this configuration, when the stent is expanded from the state of being radially contracted, the contracting force of the elastic threads causes a force that spreads the stent outward to be exerted on the vicinity of each end portion of the stent, and therefore, the end portions of the stent can be reliably expanded. Since the elastic threads are fixed to the stent, the expansion can be achieved in an extremely simple manner without needing to use a special member.

Preferably, each elastic thread is fixed to a constituent thread of the braid to form a fixing point, or crosses (is allowed to pass under) a constituent thread of the braid to form a contact point, at a position closer to the middle than an end portion of the stent. This configuration ensures that, even when the stent in a bent state is inserted into a living body, the end portions of the stent expand reliably. It is preferable that the above-described fixing point or contact point is located at a distance of 1/8 to 1/2 of the stent length from the end portion of the stent. This configuration ensures that the end portions of the stent expand more reliably.

It is preferable that the fixation of the elastic threads to the stent is performed through joining using at least one selected from thermal fusion bonding, ultrasonic fusion bonding, an adhesive, a metal fixing member, and a resin fixing member. Thermal fusion bonding and ultrasonic fusion bonding make it relatively easy to perform the fixing operation. One end of each elastic thread is fixed to the vicinity of an end portion of the stent. With regard to this fixation, although it is possible to fix the end of the elastic thread to the extreme end portion of the stent, the expansion of the end potion of the stent can be more effectively achieved by fixing the end of the elastic thread to a braid intersection, where filament threads cross each other, near the end portion of the stent. This braid intersection to which the end of the elastic thread is fixed is not limited to the extreme end portion, and may be any of the first to about third braid intersections from the extreme end portion. Moreover, the fixing point to which the other end of the elastic thread is fixed can be changed depending on the length of the elastic thread that is used, and if the elastic thread has approximately the same length as the stent after the expansion, and the other end of the elastic thread is fixed to the vicinity of the other end portion of the stent, both end portions of the stent can be expanded with a single elastic thread. Examples of the aforementioned fixing members include metal or resin tubes. In the case where a metal or resin tube is used, the joining is achieved through crimping using a C-shaped or O-shaped tube, for example.

As another fixation method, the elastic threads can be tied to constituent filament threads of the stent. With this method, the fixation can be performed in a simple manner without needing to use a special member for fixation.

According to the simplest embodiment of the present invention, each elastic thread is disposed outside a stent, and the two ends of the elastic thread are fixed only to the vicinities of the two end portions of the stent. In this case, the contracting force of the elastic threads is exerted only between the two end portions of the stent, which may result in bending of the stent. As a method for addressing this issue, it is preferable to position the contact points between each elastic thread and the stent at a distance of 1/8 to 1/2 of the stent length from the corresponding end portions of the stent.

Although a contact point between an elastic thread and the stent can be formed using the above-described fixation methods, it is also possible that an elastic thread crosses a filament thread constituting the stent. The number of contact points may be one, or two or more. In the case where two or more contact points are formed, the elastic thread between the contact points may be disposed outside or inside the stent, or may be braided into the stent.

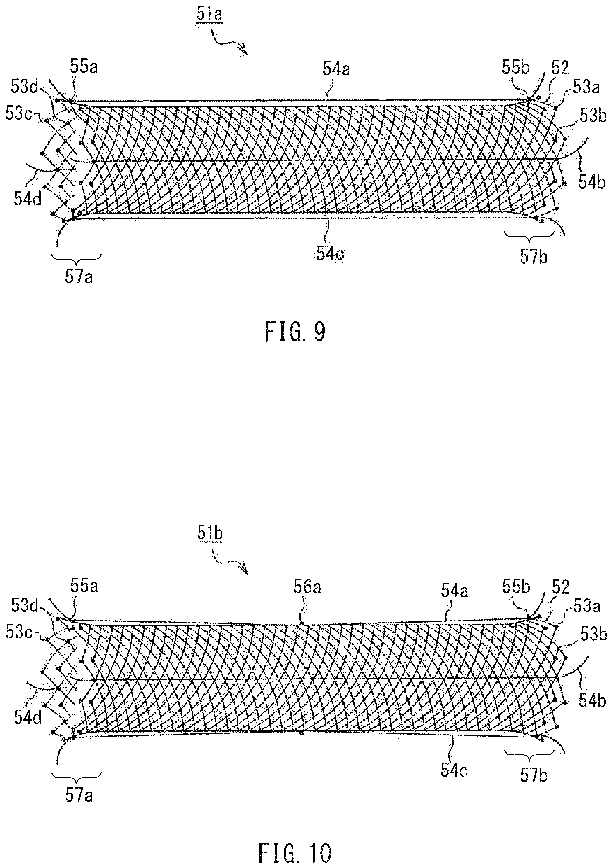

FIG. 9 is a schematic side view of a biodegradable stent 51a according to an embodiment of the present invention. The stent 51a is constituted by filament threads 52 of a braid constituting the stent, as well as elastic threads 54a-54d that are disposed outside the stent. In this example, one end of each of the elastic threads 54a-54d is fixed to the vicinity of an end portion of the stent, while the other end is fixed to another end portion of the stent, and reference numerals 55a and 55b denote fixing points of the elastic thread. The fixation is achieved through fusion bonding. In this stent, in a state in which the stent is radially contracted, tension is applied to the elastic threads 54a-54d. Note that, at the two end portions of the stent, ends of the filament threads 52 of the braid are fixed to each other through fusion bonding or the like. Reference numeral 53a denotes a fixing point at a tip of a filament thread 52. At one end portion 57a of the stent, connecting points 53a and 53b of filament thread end portions are arranged in two rows in the length direction. At the other end portion 57b of the stent as well, connecting points 53c and 53d of filament thread end portions are arranged in two rows in the length direction.

FIG. 10 is a schematic side view of a stent 51b according to another embodiment of the present invention, in which contact points where elastic threads are in contact with constituent threads of the stent are located at a distance of 1/2 of the stent length from the end portions of the stent. The difference from FIG. 9 is that the elastic thread 54a crosses (is allowed to pass under) a filament thread at a middle portion of the stent and forms a contact point 56a. The contact point 56a may also be made as a fixing point. With this configuration, even when the stent in a bent state is inserted into a living body, the end portions of the stent expand properly.

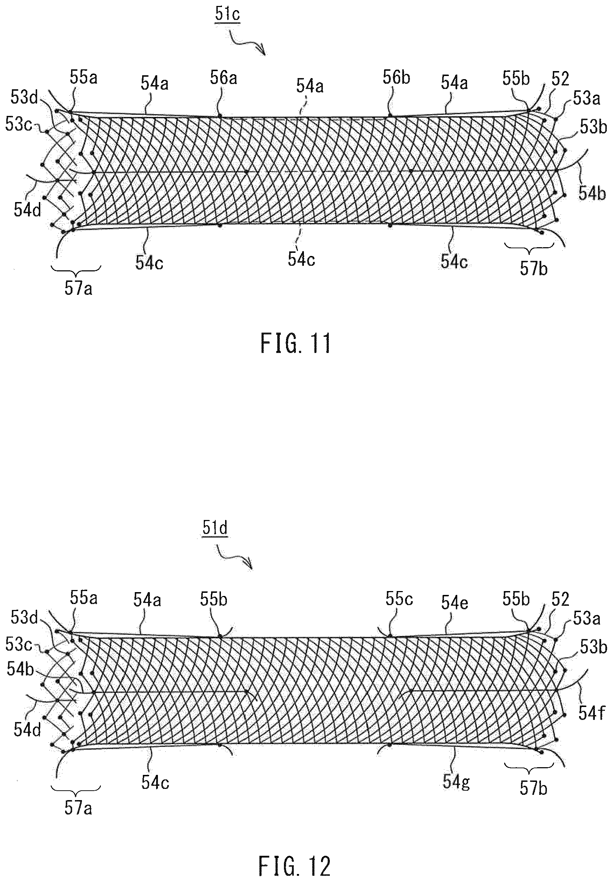

FIG. 11 is a schematic side view of a stent 51c according to yet another embodiment of the present invention, in which contact points where elastic threads are in contact with constituent threads of the stent are located at a distance of 1/3 of the stent length from the respective end portions of the stent, and the elastic threads between contact points 56a and 56b are disposed inside the stent. The contact points 56a and 56b may also be made as fixing points. With this configuration, as is the case with FIG. 10, even when the stent in a bent state is inserted into a living body, the end portions of the stent expand properly.

FIG. 12 is a schematic side view of a stent 51d according to yet another embodiment of the present invention, in which elastic threads are disposed outside the vicinities of the end portions of the stent. An elastic thread 54a is fixed to filaments constituting the stent at fixing points 55a and 55b, and an elastic thread 54b is fixed to filaments constituting the stent at fixing points 55c and 55d. With this configuration, at least the two end portions will expand outward.

When a stent of the present invention is expanded from a state of being radially contracted, the rate of change relative to the original average diameter is preferably within a range of -15% to +30%, and more preferably within a range of -10% to +25%, in the length direction. With this configuration, the stent can self-expand uniformly, and can, in particular, self-expand to a state close to the original state.

When a stent of the present invention is expanded from a state in which the stent is loaded in a delivery system to a released state (no-load state), the stent is expanded in the diameter direction by a factor of preferably greater than 5, and more preferably 6 or greater. An expansion factor within the above-described range is convenient for the insertion of the stent into a tubular portion of a living body.

When a stent of the present invention is expanded and allowed to stand in a no-load state, the stent preferably has an outer diameter of 1 to 40 mm and a length of 5 to 200 mm. In the case of a stent that is intended to be applied to the intestinal tract, the outer diameter of the stent is preferably 2 to 40 mm, more preferably 5 to 30 mm, and even more preferably 10 to 25 mm. If the outer diameter is within the above-described range, the intestinal tract of a living body can be sufficiently expanded. Moreover, a configuration may also be adopted in which the stent diameter during the loading into the delivery system is 2.3 mm or less, and the stent diameter after the expansion is 18 mm or greater. With this configuration, it is easy to perform the insertion and the placement of the stent into a living body with the use of an endoscope or the like that is distributed on the market.

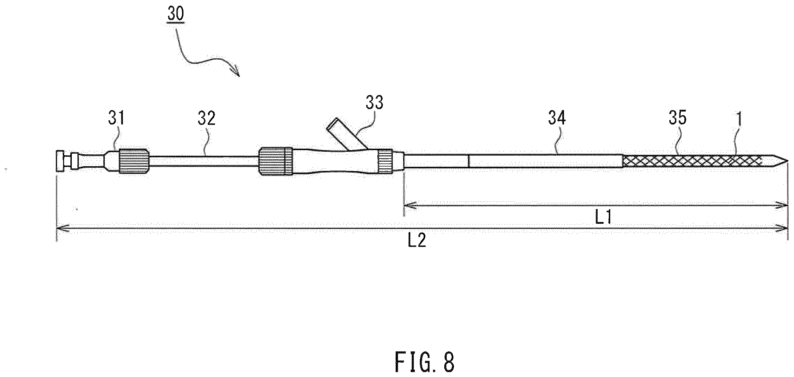

FIG. 8 is a schematic side view of a delivery system 30 into which a stent 1 of an embodiment of the present invention is loaded. This system includes a hub 31, a pusher 32, a Y-connector 33, and an outer sheath 34 that has an inner catheter inside, and the stent 1 is loaded into a stent loading portion 35 of the inner catheter. In this state, the system is inserted into a living body.

### Examples

Hereinafter, the present invention will be described in greater detail using examples and comparative examples below. However, the present invention is not limited to the following examples.

### Example 1

Polyglycolic acid with an inherent viscosity of 1.51 dL/g (0.1 g/dL HFIP, 25°C) was used as the biodegradable polymer, melt-spun at a temperature of 190°C to 245°C, drawn to a draw ratio of 4 to 5, and heat-set at a temperature of 100°C to 120°C. The obtained monofilament threads had a diameter of 0.265 mm. A stent shown in FIG. 4 was produced using the obtained monofilaments and a braid making apparatus (note that the number of carriers of the braid making apparatus was 32) shown in FIGS. 6 and 7. At the two end portions of this stent, threads were fusion-bonded to each other through ultrasonic fusion bonding, solidified, and thus connected so that connecting points were arranged in two rows in the length direction.

This stent had an outer diameter of 20 mm in an expanded state. Warp threads each constituted by a bundle of three polyurethane elastic threads manufactured by Lubrizol, U.S.A., under the brand name "Pellethane" (diameter: 70 µm) were inserted into the stent at four positions that were spaced at regular intervals around the circumference of the stent. Moreover, the braid angle θ, shown in FIG. 5, of the monofilament threads constituting the braid was set at 55°. This stent was loaded into a delivery system shown in FIG. 8, which had an outer sheath with an inner diameter of 2.5 mm. The stent was easily loaded by hand without needing a jig. Moreover, when the stent was pushed out of the delivery system, the stent self-expanded to an outer diameter of 20 mm, which means that the stent expanded by a factor of 7.1.

### Comparative Example 1

Similar procedures to those of Example 1 were performed except that, at the two end portions of the stent, threads were fusion-bonded to each other through ultrasonic fusion bonding and solidified so that connecting points were formed in a single row.

The obtained stent was loaded into a delivery system shown in FIG. 8. The loading required a jig and was difficult.

### Example 2

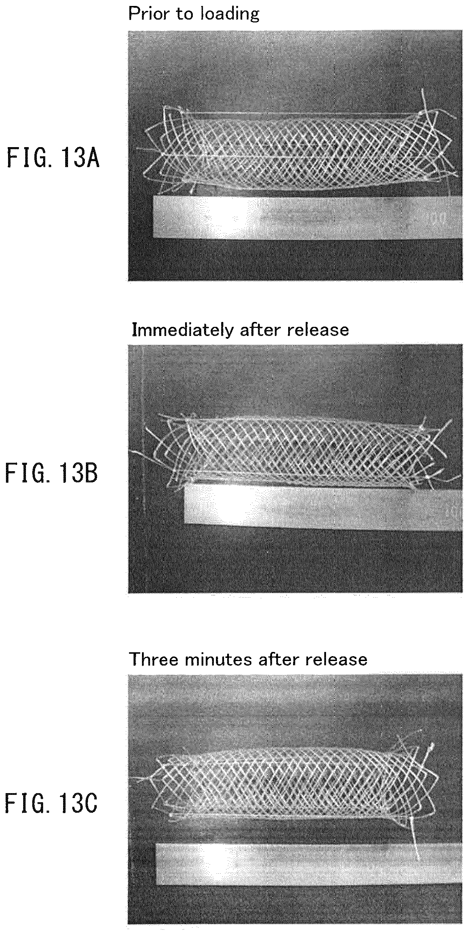

A braided stent (diameter: 22 mm, length: 80 mm) was produced using 32 monofilament threads (diameter: 0.23 mm) made of polyglycolic acid and a braid making apparatus. Polyurethane elastic threads (manufactured by Lubrizol, U.S.A., under the brand name "Pellethane" (diameter: 200 µm)) were disposed outside this braided stent as shown in FIG. 9, and fixed to the two end portions of the stent. Note however that, at the two ends of the stent, fixing points were arranged in two rows in the length direction. Four polyurethane elastic threads were disposed at respective positions that were spaced at regular intervals around the circumference of the stent. This braided stent was loaded into a delivery system shown in FIG. 8, which had an inner diameter of 3 mm, and then pushed out of the delivery system. The stent self-expanded: a middle portion of the stent expanded to the original diameter of 22 mm, and the two end portions of the stent expanded to a diameter of 22 to 27 mm, which was greater than the original diameter. FIG. 13A shows a photograph showing a lateral side of the stent prior to the loading into the delivery system; FIG. 13B shows a photograph showing a lateral side of the stent immediately after the release from the delivery system; and FIG. 13C shows a photograph showing a lateral side of the stent three minutes after the release from the delivery system. It was confirmed that the stent of the present example self-expanded uniformly in a short period of rime.

### Example 3

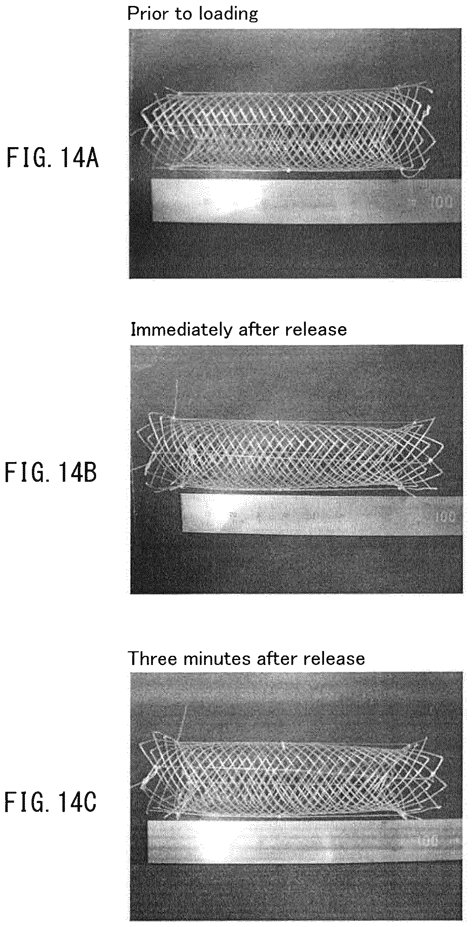

A braided stent was produced in a similar manner to that of Example 2 except that the polyurethane elastic threads crossed monofilament threads constituting the braid at the middle of the stent as shown in FIG. 10. This braided stent was loaded into a delivery system having an inner diameter of 3 mm and then pushed out of the delivery system. The stent self-expanded: a middle portion of the stent expanded to the original diameter of 22 mm, and the two end portions of the stent expanded to a diameter of 22 to 27 mm, which was greater than the original diameter. FIG. 14A shows a photograph showing a lateral side of the stent prior to the loading into the delivery system; FIG. 14B shows a photograph showing a lateral side of the stent immediately after the release from the delivery system; and FIG. 14C shows a photograph showing a lateral side of the stent three minutes after the release from the delivery system. It was confirmed that the stent of the present example self-expanded uniformly in a short period of time.

### Example 4

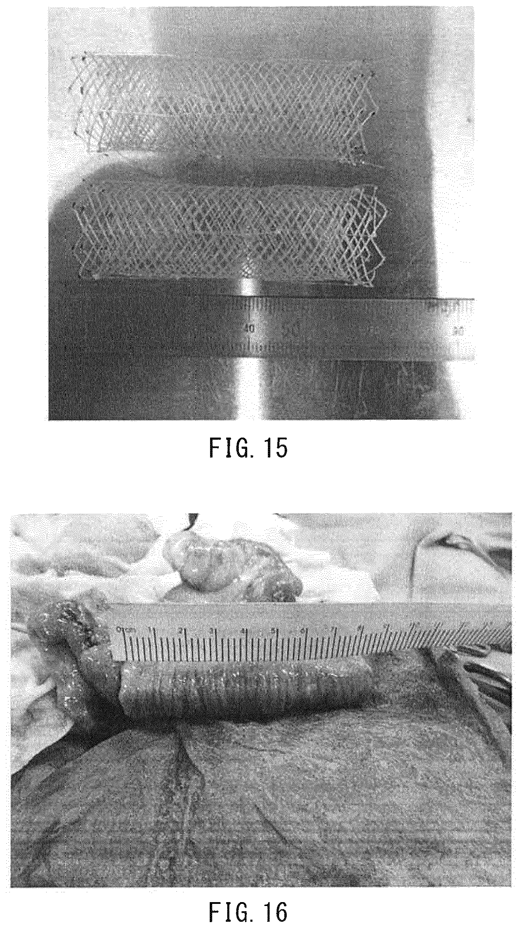

A braided stent (diameter: 20 mm, length: 65 mm) was produced using 32 monofilament threads (diameter: 0.32 mm) made of polyglycolic acid and a braid making apparatus. Polyurethane elastic threads (manufactured by Lubrizol, U.S.A., under the brand name "Pellethane" (diameter: 200 µm)) were disposed on the braided stent as shown in FIG. 11 so that contact points where the elastic threads were in contact with constituent threads of the stent were positioned, on the braid constituting the stent, at a distance of 1/3 of the stent length from the two end portions of the stent, with the elastic threads between the two end portions and the contact points extending outside the stent and the elastic threads between the contact points extending inside the braid constituting the stent. This stent was surgically placed in the small intestine of a miniature pig with the use of a delivery system having an inner diameter of 9 mm. FIG. 15 shows an external appearance of the stent of the present example prior to the placement, and FIG. 16 shows an external appearance eleven minutes after the placement into the small intestine of the miniature pig. The diameter of a middle portion was 18 mm, the diameter of the stomach-side end portion was 20 mm, and the diameter of the anus-side end portion was 16 mm. The stent was favorably expanded entirely, even to its end portions.

### Industrial Applicability

A braided stent of the present invention is suitable for insertion into a tubular portion of a living body, such as human bodies, pets, livestock, and the like, and is particularly suitable for gastrointestinal stents.

### List of Reference Numerals

- 1, 40, 51a-51d: Stent

- 2, 3: Braiding thread
- 4a, 4b: End portion
- 5a-5d: Warp thread
- 6: Length direction of braid as a whole
- 8: Braid angle
- 10: Braid making apparatus
- 11: Mount
- 12: Bobbin
- 13: Thread
- 14: Mandrel
- 15: Cylinder portion
- 16: Thrust-up portion
- 17: Braid
- 18: Guide (Pulley)
- 19: Track
- 20: Warp thread
- 21, 21a to 21d: Pipe
- 22: Elastic thread reel
- 30: Delivery system
- 31: Hub
- 32: Pusher
- 33: Y-connector
- 34: Outer sheath
- 35: Stent loading portion
- 41-48: Connecting point
- 52: Filament thread constituting braid
- 53a-53d: Fixing point at end of filament thread
- 54a-54g: Elastic thread
- 55a-55d: Fixing point where elastic thread is fixed to filament thread
- 56a-56b: Contact point or fixing point where elastic thread is fixed to or in contact with filament thread
- 57a, 57b: End portion of stent

## Claims

1. A stent (1; 40; 51a - 51d) which is formed by braiding a plurality of filament threads (2, 3) containing a biodegradable polymer into a cylindrical braid (17), wherein connecting points (42 - 48) at end portions (4a, 4b) of the filament threads constituting the braid are arranged in two or more rows in a length direction (6) of the braid, and a plurality of elastic threads (5a - 5d; 54a - 54g) are further incorporated in the braid as warp threads and extend in the length direction, with both ends of the elastic threads being fixed to the braid.

2. The stent (1; 40; 51a - 51d) according to claim 1, wherein the connecting points are formed using at least one connecting method selected from thermal fusion bonding, ultrasonic fusion bonding, and joining with the use of one of an adhesive, a metal tube, and a resin tube.

3. The stent (1; 40; 51a - 51d) according to claim 1 or 2, wherein each of the filament threads constituting the braid is a monofilament thread.

4. The stent (1; 40; 51a - 51d) according to any one of claims 1 to 3, wherein the braid is constituted by 16 or more filament threads.

5. The stent (51a - 51d) according to any one of claims 1 to 4, wherein the elastic threads (54a - 54g) are disposed outside at least a part of the stent and along at least a part of the stent in the length direction including the vicinity of either one of end portions of the stent,
an end of the elastic thread is fixed (55a - 55d) to the vicinity of the end portion of the stent, and another end of the elastic thread is fixed (56a, 56b) to any portion of the stent, and
in a state in which the stent is radially contracted, tension is applied to the elastic threads.

6. The stent (1; 40; 51a - 51d) according to claim 1 or 5, wherein the elastic threads are threads made of at least one selected from polyurethane, a rubber, and a thermoplastic elastomer that are biocompatible.

7. The stent (51b; 51c) according to any one of claims 1, 5, and 6, wherein each of the elastic threads is fixed to or crosses a constituent thread of the braid to form a fixing point or a contact point (56a, 56b) at a position that is closer to the middle than an end portion (57a, 57b) of the stent.

8. The stent (51b; 51c) according to claim7, wherein the fixing point or the contact point (56a, 56b) where the elastic thread is fixed to or crosses the constituent thread of the stent is located at a distance of 1/8 to 1/2 of a stent length from the end portion (57a, 57b) of the stent.

9. The stent (1; 40; 51a - 51d) according to any one of claims 1 and 5 to 8, wherein tension applied to the elastic threads in a state in which the stent is radially contracted is 0.1 to 5.0 N/thread.

10. The stent (1; 40; 51a - 51d) according to any one of claims 1 and 5 to 9, wherein three to six of said elastic threads are arranged at regular intervals in a circumferential direction when viewed in a cross section of the stent.

11. The stent (1; 40; 51a - 51d) according to any one of claims 1 and 5 to 10, wherein the elastic threads (5a - 5d; 54a - 54g) are fixed to the stent through joining using at least one selected from thermal fusion bonding, ultrasonic fusion bonding, an adhesive, a metal fixing member, and a resin fixing member, or tying of the elastic threads to constituent filament threads of the stent.

12. A medical device comprising the stent (1; 40; 51a - 51d) according to any one of claims 1 to 11.

13. The medical device according to claim 12, further comprising a delivery system (30).

14. The medical device according to claim 13, wherein the stent (1; 40; 51a - 51d) according to any one of claims 1 to 11 is loaded into the delivery system (30).

15. The medical device of claim 14, wherein, when the stent (1; 40; 51a - 51d) is taken out of the delivery system (30), the stent expands by an expansion factor of greater than 5 in a diameter direction.

## Patentansprüche

1. Stent (1; 40; 51a - 51d), welcher durch Flechten mehrerer ein bioabbaubares Polymer enthaltende Filament-Fäden (2, 3) zu einem zylindrischen Zopf (17) erhalten wird,
wobei Verbindungspunkte (42 - 48) an Endbereichen (4a, 4b) der den Zopf darstellenden Filament-Fäden in zwei oder mehr Reihen in einer Längsrichtung (6) des Zopfes angeordnet sind, und ferner mehrere elastische Fäden (5a - 5d; 54a - 54g) als Kettfäden in den Zopf eingearbeitet sind und sich, mit beiden Enden der elastischen Fäden am Zopf befestigt, in der Längsrichtung erstrecken.

2. Stent (1; 40; 51a - 51d) gemäß Anspruch 1, wobei die Verbindungspunkte mittels wenigstens eines Verfahrens ausgewählt aus thermischem Fusions-Bonden, Ultraschall-Fusions-Bonden und Verbinden unter Verwendung eines Klebstoffs, einer Metallröhre und einer Harzröhre verbunden werden.

3. Stent (1; 40; 51a - 51d) gemäß Anspruch 1 oder 2, wobei jeder der den Zopf darstellenden Filament-Fäden ein Monofilament-Faden ist.

4. Stent (1; 40; 51a - 51d) gemäß einem der Ansprüche 1 bis 3, wobei der Zopf aus 16 oder mehr Filament-Fäden gebildet ist.

5. Stent (51a - 51d) gemäß einem der Ansprüche 1 bis 4, wobei die elastischen Fäden (54a - 54g) außerhalb wenigstens eines Teils des Stents und entlang wenigstens eines Teils des Stents in der Längsrichtung einschließlich der Umgebung eines der Endbereiche des Stents angeordnet sind,
ein Ende des elastischen Fadens (55a - 55d) in der Umgebung des Endbereichs des Stents befestigt ist, und ein anderes Ende des elastischen Fadens (56a, 56b) in irgendeinem Bereich des Stents befestigt ist, und
in einem Zustand in welchem der Stent radial kontrahiert ist, auf die elastischen Fäden ein Zug ausgeübt wird.

6. Stent (1; 40; 51a - 51d) gemäß Anspruch 1 oder 5, wobei die elastischen Fäden solche Fäden sind, die aus wenigstens einem ausgewählt aus Polyurethan, einem Kautschuk und einem thermoplastischen Elastomer gebildet sind, die biokompatibel sind.

7. Stent (51b; 51c) gemäß einem der Ansprüche 1, 5 und 6, wobei jeder der elastischen Fäden an einem konstituierenden Faden des Zopfes befestigt ist oder jenen kreuzt, um einen Befestigungspunkt oder Kontaktpunkt (56a, 56b) an einer Stelle zu bilden, die näher zur Mitte als zu einem Endbereich (57a, 57b) des Stents liegt.

8. Stent (51b; 51c) gemäß Anspruch 7, wobei der Befestigungspunkt oder Kontaktpunkt (56a, 56b), wo der elastische Faden am konstituierenden Faden des Zopfes befestigt ist oder jenen kreuzt, in einem Abstand von 1/8 bis 1/2 der Stentlänge von einem Endbereich (57a, 57b) des Stents liegt.

9. Stent (1; 40; 51a - 51d) gemäß einem der Ansprüche 1 und 5 bis 8, wobei der Zug, der auf die elastischen Fäden in dem Zustand ausgeübt wird, in welchem der Stand radial kontrahiert ist, 0,1 bis 5.0 N/Faden beträgt.

10. Stent (1; 40; 51a - 51d) gemäß einem der Ansprüche 1 und 5 bis 9, wobei drei bis sechs der elastische Fäden in regelmäßigen Abständen in Umfangsrichtung, betrachtet im Querschnitt des Stents, angeordnet sind.

11. Stent (1; 40; 51a - 51d) gemäß einem der Ansprüche 1 und 5 bis 10, wobei die elastischen Fäden (5a - 5d; 54a - 54g) durch Verbinden mittels wenigstens einem ausgewählt aus thermischem Fusions-Bonden, Ultraschall-Fusions-Bonden, einem Klebstoff, einem Metallbefestigungsbauteil oder einem Harzbefestigungsbauteil oder durch Binden der elastischen Fäden an konstituierende Fäden des Stents an dem Stent befestigt sind.

12. Medizinische Vorrichtung mit dem Stent (1; 40; 51a - 51d) gemäß einem der Ansprüche 1 bis 11.

13. Medizinische Vorrichtung gemäß Anspruch 12, ferner aufweisend ein Kathetersystem (30).

14. Medizinische Vorrichtung gemäß Anspruch 13, wobei der Stent (1; 40; 51a - 51d) gemäß einem der Ansprüche 1 bis 11 in das Kathetersystem (30) geladen wird.

15. Medizinische Vorrichtung nach Anspruch 14, wobei der Stent (1; 40; 51a - 51d), wenn er aus dem Kathetersystem (30) genommen wird, um einen Expansionsfaktor von größer als 5 in der Durchmesser-Richtung expandiert.

## Revendications

1. Stent (1 ; 40 ; 51a - 51d) qui est formé par tressage d'une pluralité de fils de filament (2, 3) contenant un polymère biodégradable en une tresse cylindrique (17), dans lequel des points de jonction (42-48) à des parties d'extrémité (4a, 4b) des fils de filament constituant la tresse sont agencés en deux rangées ou plus dans une direction de longueur (6) de la tresse, et une pluralité de fils élastiques (5a-5d ; 54a-54g) sont en outre incorporés dans la tresse comme fils de chaîne et s'étendent dans la direction de longueur, avec les deux extrémités des fils élastiques fixées à la tresse.

2. Stent (1 ; 40 ; 51a - 51d) selon la revendication 1, dans lequel les points de jonction sont formés en utilisant au moins un procédé de jonction sélectionné parmi la liaison par fusion thermique, la liaison par fusion par ultrasons, et l'assemblage à l'aide d'un parmi un adhésif, un tube métallique et un tube en résine.

3. Stent (1 ; 40 ; 51a - 51d) selon la revendication 1 ou 2, dans lequel chacun des fils de filament constituant la tresse est un fil monofilament.

4. Stent (1 ; 40 ; 51a - 51d) selon l'une quelconque des revendications 1 à 3, dans lequel la tresse est constituée par 16 fils de filament ou plus.

5. Stent (51a - 51d) selon l'une quelconque des revendications 1 à 4, dans lequel les fils élastiques (54a-54g) sont disposés à l'extérieur d'une partie au moins du stent et le long d'une partie au moins du stent dans la direction de longueur y compris au voisinage de l'une ou l'autre des parties d'extrémité du stent,
une extrémité du fil élastique est fixée (55a-55d) au voisinage de la partie d'extrémité du stent, et une autre extrémité du fil élastique est fixée (56a, 56b) à une partie quelconque du stent, et
dans un état dans lequel le stent est contracté radialement, une tension est appliquée aux fils élastiques.

6. Stent (1 ; 40 ; 51a - 51d) selon la revendication 1 ou 5, dans lequel les fils élastiques sont des fils faits d'au moins un matériau sélectionné parmi le polyuréthanne, un caoutchouc, et un élastomère thermoplastique qui sont biocompatibles.

7. Stent (51b ; 51c) selon l'une quelconque des revendications 1, 5, et 6, dans lequel chacun des fils élastiques est fixé à un fil constitutif de la tresse ou croise celui-ci pour former un point de fixation ou un point de contact (56a, 56b) à une position qui est plus proche du milieu qu'une partie d'extrémité (57a, 57b) du stent.

8. Stent (51b ; 51c) selon la revendication 7, dans lequel le point de fixation ou le point de contact (56a, 56b) où le fil élastique est fixé au fil constitutif du stent ou croise celui-ci est situé à une distance de 1/8 à 1/2 d'une longueur de stent depuis la partie d'extrémité (57a, 57b) du stent.

9. Stent (1 ; 40 ; 51a-51d) selon l'une quelconque des revendications 1 et 5 à 8, dans lequel une tension appliquée aux fils élastiques dans un état dans lequel le stent est contracté radialement est de 0,1 à 5,0 N/fil.

10. Stent (1 ; 40 ; 51a-51d) selon l'une quelconque des revendications 1 et 5 à 9, dans lequel trois à six desdits fils élastiques sont agencés à intervalles réguliers dans une direction circonférentielle, vus en section transversale du stent.

11. Stent (1 ; 40 ; 51a - 51d) selon l'une quelconque des revendications 1 et 5 à 10, dans lequel les fils élastiques (5a-5d ; 54a-54g) sont fixés au stent par assemblage en utilisant au moins un procédé sélectionné parmi la liaison par fusion thermique, la liaison par fusion par ultrasons, un adhésif, un organe de fixation métallique, et un organe de fixation en résine, ou nouage des fils élastiques à des fils de filament constitutifs du stent.

12. Dispositif médical comprenant le stent (1 ; 40 ; 51a-51d) selon l'une quelconque des revendications 1 à 11.

13. Dispositif médical selon la revendication 12, comprenant en outre un système de distribution (30).

14. Dispositif médical selon la revendication 13, dans lequel le stent (1 ; 40 ; 51a - 51d) selon l'une quelconque des revendications 1 à 11 est chargé dans le système de distribution (30).

15. Dispositif médical selon la revendication 14, dans lequel quand le stent (1 ; 40 ; 51a - 51d) est pris dans le système de distribution (30), le stent se dilate d'un facteur de dilatation supérieur 5 dans un direction de diamètre.
